(19) Europäisches Patentamt
European Patent Office
Office européen des brevets

(11) Veröffentlichungsnummer: **0 240 867 B1**

# (12) EUROPÄISCHE PATENTSCHRIFT

(45) Veröffentlichungstag der Patentschrift: **19.02.92**

(51) Int. Cl.⁵: **C07D 251/70**, C08G 59/50

(21) Anmeldenummer: **87104554.8**

(22) Anmeldetag: **27.03.87**

Die Akte enthält technische Angaben, die nach dem Eingang der Anmeldung eingereicht wurden und die nicht in dieser Patentschrift enthalten sind.

## (54) Verfahren zur Herstellung von Aminoalkyl- oder -aryl-Melaminen.

(30) Priorität: **05.04.86 DE 3611420**

(43) Veröffentlichungstag der Anmeldung:
**14.10.87 Patentblatt 87/42**

(45) Bekanntmachung des Hinweises auf die Patenterteilung:
**19.02.92 Patentblatt 92/08**

(84) Benannte Vertragsstaaten:
**CH DE FR GB LI**

(56) Entgegenhaltungen:
**EP-A- 0 079 037**
**EP-A- 0 151 927**
**EP-A- 0 179 408**
**EP-A- 0 196 275**
**US-A- 2 393 755**
**US-A- 4 446 280**

**CHEMICAL ABSTRACTS, Band 58, Nr. 5, 04 März 1963, Columbus, OH (US); I.HONDA, Spalte 4568**

**LEHRBUCH DER ANORGANISCHEN CHEMIE, Holleman-Wiberg; S. 113f**

**ULLMANNs ENZYKLOPÄDIE DER TECHNISCHEN CHEMIE, 3. Aufl., 1960, Band 12; S. 279**

(73) Patentinhaber: **BASF Aktiengesellschaft**
**Carl-Bosch-Strasse 38**
**W-6700 Ludwigshafen(DE)**

(72) Erfinder: **Ebel, Klaus, Dr.**
**Ungsteiner Str. 18**
**W-6700 Ludwigshafen(DE)**
Erfinder: **Reuther, Wolfgang, Dr.**
**Am Pferchelhang 16**
**W-6900 Heidelberg(DE)**

EP 0 240 867 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Aminoalkyl-, Aminocycloalkyl- oder Aminoaryl-Melaminen, wobei die Verfahrensprodukte weniger als 10 Gew.-%, bezogen auf das Gesamtgewicht der Endprodukte, Nebenprodukte enthalten.

Es ist bekannt, die Aminogruppen des Melamins durch verschiedenartige Amine, beispielsweise Alkylamine, Arylamine und Alkanolamine, auszutauschen, vgl. US-A-2 328 961, US-A-2 393 755, US-A-2 467 523 und US-A-2 544 071.

In der US-A-4 446 280 sind eine Reihe von Aminoalkyl- oder Aminoaryl-Melaminen genannt. Diese fallen aber immer als unübersichtliche Gemische an, eine Auftrennung in die Einzelkomponenten wird weder beschrieben noch nahegelegt.

In der europäischen Patentanmeldung EP-A-179 408 ist die Synthese von Aminoalkylmelaminen aus Cyanurchlorid bzw. Diaminochlortriazin und einem Diamin unter Zuhilfenahme der Schutzgruppentechnik beschrieben. Dazu wird zuerst aus dem Diamin ein durch eine Schutzgruppe einseitig blockiertes Diamin hergestellt, dieses dann mit dem Chlortriazin umgesetzt und schließlich die Schutzgruppe wieder abgespalten.

Weiterhin ist aus der EP-A 151 927 ein Verfahren zur Herstellung von Melaminderivaten bekannt, in dem man Cyanurchlorid mit einem N-substituierten Aminonitril oder zunächst mit einem Alkyl-, Cycloalkyl- oder Arylamin und danach mit Acrylnitril oder einem $\alpha$-Alkylarylnitril umsetzt und anschließend die Nitrilgruppen zu Aminogruppen hydriert. Tris-N-monosubstituierte Melaminderivate mit endständigen Aminogruppen an den Substituenten sind nach diesem Verfahren jedoch nicht zugänglich.

Der Erfindung liegt die Aufgabe zugrunde, eine neues Verfahren zur Herstellung von Aminoalkyl-, cycloalkyl- oder aryl-Melaminen zur Verfügung zu stellen, das in einfacher Weise durchgeführt werden kann und das zu hohen Produktausbeuten führt.

Diese Aufgabe wird gelöst durch ein Verfahren zur Herstellung von Aminoalkyl-, Aminocycloalkyl- oder Aminoarylmelaminen der allgemeinen Formel I

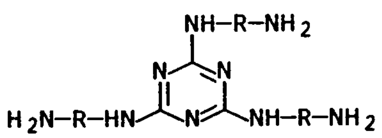

(I)

worin R für einen linearen Alkylenrest mit mindestens 2 Kohlenstoffatomen, einen verzweigten Alkylenrest mit mindestens 3 Kohlenstoffatomen, einen Cycloalkylenrest mit mindestens 5 Kohlenstoffatomen, wobei in den linearen oder verzweigten Alkylenresten oder dem Cycloalkylenrest ein oder mehrere Kohlenstoffatome durch Heteroatome ersetzt sein können, einen aromatischen Rest oder für einen Rest der allgemeinen Formel II

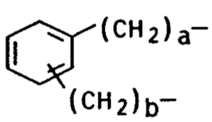

(II),

worin unabhängig voneinander a 1 bis 6 und b 0 bis 6 sein können steht, wobei die Verfahrensprodukte weniger als 10 Gew.-%, bezogen auf das Gesamtgewicht der Endprodukte, Nebenprodukte enthalten, das dadurch gekennzeichnet ist, daß man das Melamin mit einem Diamin der allgemeinen Formel III

$H_2N\text{-}R\text{-}NH_2$ (III),

worin R die obigen Bedeutungen besitzt, im Molverhältnis Diamin : Melamin ≧20 : 1 umsetzt.

Die Aminoalkyl-, Aminocycloalkyl- oder Aminoaryl-Melamine der allgemeinen Formel I sind wertvolle Zwischenprodukte. Sie sind insbesondere zur Verwendung für Polyisocyanat-Polyadditionsreaktionen und für andere Anwendungen, z.B., als Härter für Epoxidharze, geeignet. Sie eignen sich auch als Zwischenprodukte bei der Herstellung von Pharmazeutika, insbesondere nach Alkylierung der Aminfunktionen (vgl. US-A-2 725 379) und der Herstellung von Färbereihilfsmitteln.

Beim erfindungsgemäßen Verfahren entstehen - je nach Reaktionsbedingungen - Aminoalkyl-,

Aminocycloalkyl- oder Aminoaryl-Melamin entweder in ganz reiner Form oder sie enthalten als Nebenprodukte auch Verbindungen der Formel IV in weniger als 10 %, bezogen auf das Gesamtgewicht des Endprodukts.

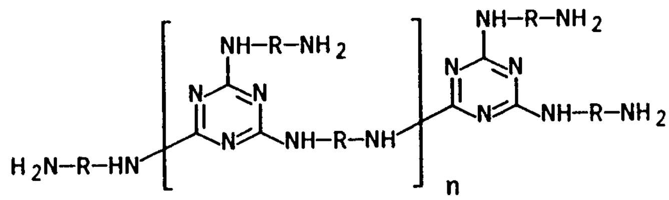

(IV)

n = 1.

Der Aminaustausch mit Diaminen, auf welchem das erfindungsgemäße Verfahren beruht, ist bislang noch nicht beschrieben worden. Das liegt wahrscheinlich daran, daß die erhaltenen monomeren Aminaustauschprodukte der Formel I in einer Selbstkondensation unter Abspaltung von Diamin zu Oligomeren und Polymeren der Formel IV/n weiterreagieren können.

Aufgrund dieser Selbstkondensation erhält man nämlich unter den gewöhnlich angewendeten Bedingungen für die Aminaustauschreaktion mit einer Diaminmenge von 3 bis 5 Mol pro Mol Melamin komplexe Produktgemische mit einem Oligomeren- bzw. Polymerenanteil von über 50 %.

überraschenderweise wurde erfindungsgemäß eine Möglichkeit gefunden, die Selbstkondensation zu verhindern und somit reine Verbindung der allgemeinen Formel I herzustellen. Entscheidend für die Reinheit der Verbindung I ist, daß die Reaktion mit einem großen Diaminüberschuß, durchgeführt wird. So erhält man mit einem Aminüberschuß, Molverhältnis Amin : Melamin von ≧20 : 1 praktisch reine Verbindung der allgemeinen Formel I. Es kann lediglich als einziges Nebenprodukt das Dimere auftreten. Die Menge an Nebenprodukt ist bei einem Molverhältnis Amin : Melamin von 20 : 1 jedoch weniger als 10 %, bezogen auf das Gesamtgewicht des Endprodukts. Bei entsprechend großem Aminüberschuß läßt sich ganz reines tris-Substitutionsprodukt herstellen.

Ganz allgemein erhält man mit steigendem Diaminüberschuß einen sinkenden Polymeren- und Oligomeren-Anteil und somit einen steigenden Anteil an monomeren Produkten.

Das erfindungsgemäße Verfahren wird zweckmäßig so durchgeführt, daß man ein Gemisch aus Melamin, Diamin (Molverhältnis Diamin : Melamin ≧20 : 1), saurem Katalysator und gegebenenfalls einem Lösungsmittel vorlegt und unter Rühren auf eine Temperatur von 120 bis 300° C, insbesondere von 160 bis 250° C erhitzt.

Diamine, deren Siedepunkt hoch genug liegt, um eine ausreichend schnelle Reaktion zu ermöglichen, werden im allgemeinen bei Normaldruck umgesetzt. Liegt der Siedepunkt des Diamins jedoch unter 160° C, so ist es vorteilhaft, die Umsetzung bei höherer Temperatur unter Druck durchzuführen. Zur Vervollständigung des Umsatzes aller $NH_2$-Gruppen des Melamins empfiehlt es sich, das gebildete Ammoniak aus der Reaktionsmischung zu entfernen. Dies geschieht beispielsweise durch Abdestillieren unter Druck von 1 bis 20 bar.

Weiterhin empfiehlt es sich, die Umsetzung in Gegenwart eines Schurtzgases durchzuführen. Das Schutzgas wird dabei im allgemeinen über die Oberfläche der Reaktionsmischung geleitet. Geeignete Schutzgase sind z.B. Edelgase und insbesondere Stickstoff.

Als saure Katalysatoren kommen alle starken und mittelstarken Protonensäuren in Betracht, z.B. Flußsäure, Amidosulfonsäure, Thiocyansäure, p-Toluolsulfonsäure oder Methansulfonsäure.

Die Säuren können entweder in freier Form oder als Melamin- oder Diaminsalz zugegeben werden. Weiterhin ist auch die Zugabe als Salz einer Base, die schwächer als das Amin ist, möglich, beispielsweise als Ammoniumsalz.

Anstelle der genannten Protonensäuren katalysieren auch andere Lewis-Säuren, wie Bortrifluorid, Aluminiumchlorid, Zink(IV)chlorid, Antimon(V)fluorid oder Eisen(III)bromid die Reaktion.

Pro Mol Melamin werden zweckmäßig 0,05 bis 3 Mol, vorzugsweise 0,1 bis 1 Mol Katalysator eingesetzt. Für den Fall, daß man die Protonensäure in Form ihres Melaminsalzes verwendet, muß die Melamin menge aus dem Salz berücksichtigt werden.

Man kann im erfindungsgemäßen Verfahren gewünschtenfalls mit organischen Lösungsmitteln arbeiten. Als organische Lösungsmittel kommen Polyole in Betracht, beispielsweise Ethylenglykol, 1,2-Propylenglykol, Diethylenglykol oder Triethylenglykol. Vorzugsweise wird das Verfahren jedoch in Abwesenheit von Lösungsmitteln durchgeführt.

Der Rest R der allgemeinen Formel I kann für einen linearen Alkylenrest mit mindestens 2 Kohlenstoffatomen stehen. Besonders bevorzugt sind lineare Alkylenreste mit 2 bis 20 Kohlenstoffatomen, insbesondere lineare Alkylenreste mit 2 bis 16 Kohlenstoffatomen, insbesondere mit 2 bis 12 Kohlenstoffatomen. Beispielhaft zu nennen sind Ethylen-, Propylen-, Butylen-, Pentylen-, Hexylenreste. R kann auch für einen verzweigten Alkylenrest mit mindestens 3 Kohlenstoffatomen stehen. Der verzweigte Alkylenrest kann beispielsweise 3 bis 20 Kohlenstoffatome, insbesondere 3 bis 15 Kohlenstoffatome aufweisen. Es kann sich beispielsweise um einen Alkylenrest handeln, der durch ein oder mehrere Niedrigalkylreste, beispielsweise Methyl, Ethyl, Propyl, substituiert ist. R kann auch für einen Cycloalkylenrest mit mindestens 5 Kohlenstoffatomen stehen, beispielsweise einen Cyclopentylenrest, einen Cyclohexylenrest, einen 1,3-Cyclopentylenrest, oder einen 1,2-Cyclohexylenrest, einen 1,3-Cyclohexylenrest, einen 1,4-Cyclohelenrest. In den linearen oder verzweigten Alkylenresten oder dem Cycloalkylenrest können ein oder mehrere Kohlenstoffatome durch Heteroatome ersetzt sein. Als Heteroatome kommen in Betracht O, S, N in Form der Gruppierung, $-NHR^1-$. Beispielsweise können die linearen oder verzweigten Alkylenreste oder der Cycloalkylenrest durch 2 oder 3 Sauerstoffatome, Schwefelatome oder Stickstoffgruppierungen unterbrochen sein. Der Rest $R^1$ in der Stickstoffgruppierung kann verschiedene Bedeutungen besitzen, beispielsweise kann er für einen Alkylrest, insbesondere einen Alkylrest mit 1 bis 6 Kohlenstoffatomen oder auch für Wasserstoff stehen.

Der Rest R kann auch für einen gegebenenfalls substituierten aromatischen Rest stehen. Hierbei kommt insbesondere ein Phenylenrest oder Naphthylenrest in Betracht, beispielsweise ein ortho-, metha- oder para-Phenylenrest. Als Substituenten für den aromatischen Rest kommen Substituenten in Betracht, die unter den Reaktionsbedingungen des erfindungsgemäße Verfahrens inert sind, beispielsweise ein oder mehrere Halogenatome, Alkylreste, Alkoxyreste, Hydroxylgruppen und äquivalente Substituenten.

Der Rest R kann auch für einen Rest der allgemeinen Formel II stehen.

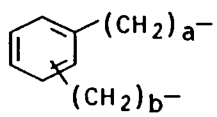

(II),

worin unabhängig voneinder a 1 bis 6 und b 0 bis 6 sein können.

Der Rest der allgemeinen Formel II kann durch beliebige Substituenten substituiert sein, insbesondere kommen dieselben Substituenten wie beim obigen aromatischen Rest in Betracht. Die Substitution kann im Kern oder gegebenenfalls in der Seitenkette vorliegen.

Entscheidender Verfahrensparameter in Bezug auf die Produktzusammensetzung ist das Molverhältnis von Diamin : Melamin. Mit zunehmendem Diaminüberschuß erhält man bei vollständigem Umsatz immer reineres I.

Dies wird am Beispiel des Aminaustauschs mit Hexamethylendiamin anhand des Gehalts an Tris-(6-aminohexyl)-melamin im Endprodukt in der nachfolgenden Tabelle quantifiziert.

| | | | | | | | | | |
|---|---|---|---|---|---|---|---|---|---|
| Molverhältnis Diamin : Melamin | 3 | 4 | 5 | 6 | 10 | 17 | 20 | 40 | 80 |
| Gehalt an Tris-(6-aminohexyl)melamin (Gew.-%) | 26 | 45 | 56 | 65 | 78 | 88 | 92 | 96 | 98 |

Die Gehalte an Tris-(6-aminohexyl)melamin wurden durch quantitative HPLC mit reiner Eichsubstanz ermittelt. Die zu 100 % fehlende Masse besteht aus Oligomeren vom Typ der Verbindung IV mit R = $(CH_2)_6$.

Herstellung der Eichsubstanz Tris-(6-aminohexyl)melamin:

1,85 g (0,01 mol) Cyanurchlorid und 10,0 g (0.04 mol) Mono-N-Carbobenzoxyhexamethylendiamin werden unter Erwärmen und Zugabe von 25 %iger Natronlauge bei pH 8,1 gehalten und zum Schluß unter Rückfluß gekocht. Man erhielt nach dem Aufarbeiten und Umkristallisieren aus Ethanol N,N',N"-Tris-(6-benzyloxycarboxylamidohexyl)melamin mit Schmp. 98 °C.

21,2 g der auf diese Weise erhältichen Verbindung (0,03 mol) werden 20 h bei 30 bar $H_2$ / Raumtemperatur in 100 ml Ethanol/100 ml Eisessig über 0,6 g Palladium auf Aktivkohle hydriert. Der Katalysator wurde abfiltriert, und das Reaktionsgemisch wurde zur Trockene im Vakuum eingedampft. Der Rückstand wurde durch Chromatographie an stark basischem Ionenaustauscher von noch anhaftendem Eisessig befreit. Man erhielt ein farbloses Öl mit folgenden Kennzeichen:

$^1H$ - NMR

| | | | |
|---|---|---|---|
| δ [ppm] = | 1,3 (s, br., 24H) | 2,0 (s, 6H) | 2,5 (m, 6H) |
| | 3,2 (m, 6H) | 6,3 (s, br., 3H) | |

| Analyse | C | H | N |
|---|---|---|---|
| Ber.: | 59,54 | 10,71 | 29,76 |
| Gef.: | 59,1 | 10,8 | 29,2 |

Mit den anderen erfindungsgemäßen Diaminen erhält man Produkte mit analoger Zusammensetzung.

Die Erfindung wird durch die nachstehenden Beispiele näher erläutert.

Beispiel 1

Tris-(6-aminohexyl)melamin

Man kochte 63,5 g (0,5 Mol) Melamin, 1162 g (10,0 Mol) Hexamethylendiamin und 26,8 g (0,5 Mol) Ammonchlorid unter Rühren und Überleiten eines schwachen Stickstoffstromes 14 - 16 h unter Rückfluß (etwa 210° C). Dann wurden bei 100° C 48 g (0,6 Mol) 50 %ige Natronlauge zugegeben und heiß vom ausgefallenen Salz abfiltriert. Schließlich wurde der Hexamethylendiaminüberschuß im Vakuum vom Filtrat abdestilliert und man erhielt 211 g (100 %) eines farblosen zähen Öls. Nach der quantitativen HPLC-Analyse enthielt das Produkt 92 Gew.-% Tris-(6-aminohexyl)melamin. Der Rest ist Verbindung IV/n = 1 mit $R = (CH_2)_6$.

Das $^1H$-NMR-Spektrum zeigte keine Unterschiede zum auf unabhängigem Weg hergestellten reinen Tris-(6-aminohexyl)melamin.

Vergleichsbeispiele A bis F und Beispiele 2 und 3

Die Beispiele 2 und 3 und Vergleichsbeispiel A bis F wurden völlig analog dem Beispiel 1 durchgeführt. Sie unterschieden sich lediglich durch das Ausgangsmolverhältnis von Diamin : Melamin.

Aus den unterschiedlichen Ausgangsmolverhältnissen resultierten Produkte mit unterschiedlicher Zusammensetzung. Der Anteil des Tris-(6-aminohexyl)-melamins nahm mit steigendem Ausgangsmolverhältnis von Diamin : Melamin zu. Ab einem Ausgangsmolverhältnis von Hexamethylendiamin : Melamin von 20 : 1 ist die Verbindung IV mit $R = (CH_2)_6$ das einzige Nebenprodukt. Bei niedrigerem Ausgangsmolverhältnis waren zunehmend höhere Oligomere IV/n mit $n \geqq 1$ und $R = (CH_2)_6$ durch HPLC nachweisbar.

Die Einwaagen der Beispiele und Vergleichsbeispiele, sowie die durch quantitative HPLC bestimmten Gehalte an Tris-(6-aminohexyl)melamin sind in der nachfolgenden Tabelle aufgeführt. Die Ausbeuten sind in allen Fällen quantitativ.

| Bsp. | Einwaage | | | | | | Produkt |
|---|---|---|---|---|---|---|---|
| | Melamin | | Ammonchlorid | | Hexamethylendiamin | | Gehalt an Tris-(6-aminohexyl)-melamin |
| Nr. | (g) | (mol) | (g) | (mol) | (g) | (mol) | (Gew.-%) |
| A | 63,0 | 0,5 | 26,8 | 0,5 | 174,3 | 1,5 | 26 |
| B | 63,0 | 0,5 | 26,8 | 0,5 | 232,4 | 2 | 45 |
| C | 63,0 | 0,5 | 26,8 | 0,5 | 290,5 | 2,5 | 56 |
| D | 63,0 | 0,5 | 26,8 | 0,5 | 348,6 | 3 | 65 |
| E | 63,0 | 0,5 | 26,8 | 0,5 | 581,0 | 5 | 78 |
| F | 63,0 | 0,5 | 26,8 | 0,5 | 1045,8 | 9 | 88 |
| 1 | 63,0 | 0,5 | 26,8 | 0,5 | 1162,0 | 10 | 92 |
| 2 | 63,0 | 0,5 | 26,8 | 0,5 | 2324,9 | 20 | 96 |
| 3 | 37,8 | 0,3 | 32,1 | 0,6 | 2788,8 | 24 | 98 |

Beispiel 4

Tris-(2-aminoethyl)melamin

Man kochte 190 g (1,5 Mol) Melamin, 1800 g (30 Mol) Ethylendiamin und 80 g (1,5 Mol) Ammonchlorid bei 200°C und 10 bar unter Rühren bis zum vollständigen Umsatz am Rückfluß. Das bei der Reaktion gebildete Ammoniak wurde über ein Regelventil aus der Reaktionslösung entfernt. Nach dem Abkühlen versetzte man mit 195 g (1,7 Mol) 50 %iger Kalilauge und filtrierte das ausgefallene Salz ab. Das überschüssige Ethylendiamin wurde im Vakuum abdestilliert und man erhielt 380 g (99 %) eines fast farblosen Harzes, welches nach der quantitativen HPLC-Analyse 90 Gew.-% Tris-(2-aminoethyl)-melamin enthielt. Der Rest ist Verbindung IV/n = 1 mit $R = (CH_2)_2$.

Das $^1$H-NMR-Spektrum zeigte keine Unterschiede zum auf unabhängigem Weg hergestellten Tris-(2-aminoethyl)melamin.

Beispiel 5

Tris-(3-aminopropyl)melamin

Man kochte 12,6 g (0,1 Mol) Melamin, 148,0 g (2,0 Mol) 1,3-Diaminopropan und 5,4 g (0,1 Mol) Ammonchlorid bei 20°C und 9 bar analog wie in Beispiel 4 bis zum vollständigen Umsatz am Rückfluß. Nach analoger Aufarbeitung wie in Beispiel 4, erhielt man 28,8 g (100 %) eines farblosen Harzes.

$^1$H-NMR-Spektrum

S/ppm = 1,5 (t, j = 9 Hz, H); 2,5 (t, j = 9 Hz, g H); 2,9 (S, 6 H); 3,2 (t, j = 9 Hz, 6 H); 6,4 (S. br., 3H)

Beispiel 6

Tris-(4-aminobutyl)melamin

Man kochte 12,6 g (0,1 Mol) Melamin, 184,0 g (2,0 Mol) 1,4-Diaminobutan und 5,4 g (0,1 Mol) Ammonchlorid bei 200°C und 8 bar analog wie in Beispiel 4 bis zum vollständigen Umsatz unter Rückfluß. Nach analoger Aufarbeitung wie in Beispiel 4 erhielt man 33,8 g (100 %) eines farblosen Harzes.

$^1$H-NMR-Spektrum

S/ppm/ = 1,3 (t, j = 9 Hz, 12 H); 9,9 (S, 6 H); 2,5 (t, j = 9 Hz, 6 H); 3,2 (t, j = 9 Hz, 6 H); 6,4 (S. br., 3 H)

Beispiel 7

Tris-(12-aminododecyl)melamin

Man erhitzte 8,4 g (0,067 Mol) Melamin, 267 g (1,34 Mol) 1,12-Diaminodocean und 3,57 g (0,067 Mol) Ammonchlorid bis zum vollständigen Umsatz unter Rühren und überleiten eines schwachen Stickstoffstromes auf 210 °C. Dann wurden bei 120 °C 11,0 g (0,069 Mol) 25 %ige Natronlauge zugegeben und das ausgefallene Salz abgesaugt. Schließlich wurde der 1,12-Diaminodeceanüberschuß im Vakuum abgestilliert und man erhielt 45,5 g (100 %) eines farblosen festen Rückstands.

$^1$H-NMR-Spektrum

S/ppm/ = 1,3 (S, 60 H); 2,6 (m, 12 H); 3,3 (T, 6 H); 5,2 (S, br., 4 H)

Beispiel 8

Tris-(10-amino-4,7-diodecyl)melamin

Man erhitzte 12,6 g (0,1 Mol) Melamin, 5,35 g (0,1 Mol) Ammonchlorid und 352,0 g (2,0 Mol) 4,7-Dioxadecan-1,10-diamin bis zum vollständigen Umsatz unter Rühren und Überleiten eines schwachen Stickstoffstromes auf 210 °C.

Dann wurden bei 120 °C 16,6 g (0,1 Mol) 25 %ige Natronlauge zugegeben und das ausgefallene Salz abgesaugt. Schließlich wurde das überschüssige 4,7-Dodecanmethylendiamin im Vakuum abdestilliert und man erhielt 60,5 g (100 %) eines fast farblosen Harzes.

$^1$H-NMR-Spektrum

S/ppm/ = 1,6 (quint., j = 9 Hz, 12 H); 2,6 (S, br., 12 H); 3,3 (t, j = 9 HZ, 6 H); 3,4 (S, 24 H); 6,4 (S, br., 3 H)

Beispiel 9

Tris-(3-aminophenyl)melamin

Man erhitzte 12,6 g (0,1 Mol) Melamin, 5,35 8 (0,1 Mol) Ammonchlorid und 217,0 g (2,0 Mol) 1,3-Phenylendiamin bis zum vollständigen Umsatz unter Rühren und Überleiten eines schwachen Stickstoffstromes auf 210 °C. Nach analoger Aufarbeitung wie in Beispiel 13, erhielt man 40,1 g (100 %) eines rotbraunen harzartigen Rückstandes.

$^1$H-NMR-Spektrum

S/ppm/ = 4,6 (s, br., 6 H); 6,0 - 7,4 (m, 12 H); 8,8 (s, br., 3 H) In diesem Beispiel läßt sich das 1,3-Phenylendiamin durch 1,3-Diaminocyclohexan ersetzen.

Beispiel 10

Tris-[3-(aminoethyl)benzyl]melamin

Man erhitzte 12,6 g (0,1 Mol) Melamin, 5,35 g (0,1 Mol) Ammonchlorid und 290,0 g (2,0 Mol) 1,3-Bis-(aminomethyl)benzol bis zum vollständigen Umsatz unter Rühren und überleiten eines schwachen Stickstoffstromes auf 210 °C. Nach analoger Aufarbeitung erhielt man 48,5 g (100 %) eines farblosen Harzes.

$^1$H-NMR-Spektrum

S/ppm/ = 2,1 (s, 6 HJ); 3,7 (s, 6 H); 4,4 (s, 6 H); 7,2 (m, 15 H)

**Patentansprüche**

**1.** Verfahren zur Herstellung von Aminoalkyl-, Aminocycloalkyl- oder Aminoaryl-Melaminen der allgemeinen Formel I

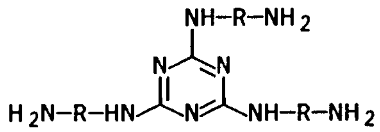

(I)

worin R für einen linearen Alkylenrest mit mindestens 2 Kohlenstoffatomen, einen verzweigten Alkylenrest mit mindestens 3 Kohlenstoffatomen, einen Cycloalkylenrest mit mindestens 5 Kohlenstoffatomen, wobei in den linearen oder verzweigten Alkylenresten oder dem Cycloalkylenrest ein oder mehrere Kohlenstoffatome durch Heteroatome ersetzt sein können, einen aromatischen Rest oder für einen Rest der allgemeinen Formel II

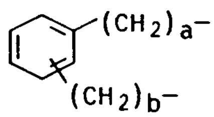

(II),

worin unabhängig voneinander a 1 bis 6 und b 0 bis 6 sein können steht, wobei die Verfahrensprodukte weniger als 10 Gew.-%, bezogen auf das Gesamtgewicht der Endprodukte, Nebenprodukte enthalten, dadurch gekennzeichnet, daß man das Melamin mit einem Diamin der allgemeinen Formel III

$H_2N\text{-}R\text{-}NH_2$ (III),

worin R die obigen Bedeutungen besitzt, im Molverhältnis Diamin : Melamin ≧20 : 1 umsetzt.

**2.** Verfahren nach Anspruch 1, dadurch gekennzeichnet, daß man das bei der Reaktion freiwerdende Ammoniak aus der Reaktionsmischung entfernt.

**3.** Verfahren nach einem der Ansprüche 1 oder 2, dadurch gekennzeichnet, daß man bei einer Temperatur zwischen 160 und 250 °C arbeitet.

**4.** Verfahren nach einem der Ansprüche 1 bis 3, dadurch gekennzeichnet, daß man unter Schutzgas arbeitet.

**5.** Verfahren nach einem der Ansprüche 1 bis 4, dadurch gekennzeichnet, daß man als sauren Katalysator Protonensäuren oder andere Lewis-Säuren einsetzt.

**6.** Verfahren nach Anspruch 5, dadurch gekennzeichnet, daß man 0,05 bis 3 Mol Katalysator pro Mol Melamin einsetzt.

**7.** Verfahren nach einem der Ansprüche 5 oder 6, dadurch gekennzeichnet, daß man als Katalysator Ammoniumchlorid verwendet.

## Claims

**1.** A process for the preparation of aminoalkyl-, aminocycloalkyl- or aminoaryl-melamines of the formula I

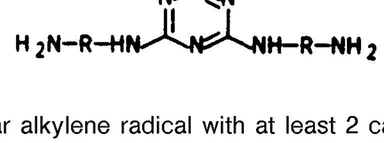

(I)

in which R is a linear alkylene radical with at least 2 carbon atoms, a branched alkylene radical with at least 3 carbon atoms, a cycloalkylene radical with at least 5 carbon atoms, it being possible for one or more carbon atoms in the linear or branched alkylene radicals or the cycloalkylene radical to be replaced by hetero atoms, or is an aromatic radical or a radical of the formula II

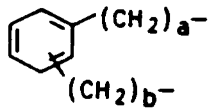

(II)

in which, independently of one another, a can be from 1 to 6 and b can be from 0 to 6, where the products of the process contain less than 10% by weight, based on the total weight of the final products, of byproducts, which comprises reacting the melamine with a diamine of the formula III

$H_2N\text{-}R\text{-}NH_2$ (III)

in which R has the above meanings, in the molar ratio diamine:melamine ≧ 20:1.

2. A process as claimed in claim 1, wherein the ammonia liberated in the reaction is removed from the reaction mixture.

3. A process as claimed in either of claims 1 or 2, which is carried out at from 160 to 250 °C.

4. A process as claimed in any of claims 1 to 3, which is carried out under protective gas.

5. A process as claimed in any of claims 1 to 4, wherein protic acids or other Lewis acids are employed as acid catalyst.

6. A process as claimed in claim 5, wherein from 0.05 to 3 mole of catalyst are employed per mole of melamine.

7. A process as claimed in either of claims 5 or 6, wherein ammonium chloride is used as catalyst.

## Revendications

1. Procédé de préparation d'aminoalkyl-, aminocycloalkyl- ou aminoaryl-mélamines de formule générale I

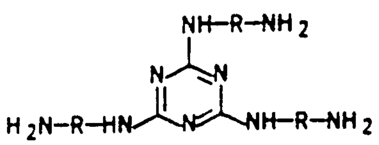

(I)

dans laquelle R représente un groupe alkylène linéaire à au moins 2 atomes de carbone, un groupe alkylène ramifié à au moins 3 atomes de carbone, un groupe cycloalkylène à au moins 5 atomes de carbone, un ou plusieurs atomes de carbone des groupes alkylène linéaires ou ramifiés ou du groupe cycloalkylène pouvant être remplacés par des hétéroatomes, un groupe aromatique ou un groupe de formule générale II

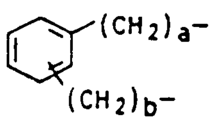

(II),

dans laquelle, indépendamment l'un de l'autre, a a une valeur de 1 à 6 et b une valeur de 0 à 6, les produits obtenus contenant moins de 10 % en poids, par rapport au poids total des produits finals, de produits d'accompagnement, caractérisé en ce que l'on fait réagir la mélamine avec une diamine de formule générale III

$H_2N-R-NH_2$ (III),

dans laquelle R a les significations indiquées cidessus, en un rapport molaire diamine/mélamine 20:1.

**2.** Procédé selon la revendication 1, caractérisé en ce que l'on élimine du mélange de réaction l'ammoniac libéré dans la réaction.

**3.** Procédé selon une des revendications 1 ou 2, caractérisé en ce que l'on opère à une température de 160 à 250 degrés C.

**4.** Procédé selon une des revendications 1 à 3, caractérisé en ce que l'on opère en atmosphère de gaz protecteur.

**5.** Procédé selon une des revendications 1 à 4, caractérisé en ce que l'on utilise en tant que catalyseurs acides des acides protoniques ou d'autres acides de Lewis.

**6.** Procédé selon la revendication 5, caractérisé en ce que l'on utilise de 0,05 à 3 moles de catalyseur par mole de mélamine.

**7.** Procédé selon une des revendications 5 ou 6, caractérisé en ce que l'on utilise le chlorure d'ammonium en tant que catalyseur.